# EUROPEAN PATENT APPLICATION

(11) **EP 2 128 118 A1**
(43) Date of publication of application: **02.12.2009**
(21) Application number: 08009687.8
(22) Date of filing: 28.05.2008
(51) Int. Cl.: C07B 35/02, C07C 45/61, C07C 49/403

(54) **Synthesis of mono-ketones from 1,3-diketones**

(71) Applicant: DSM IP Assets B.V., 6411 TE Heerlen (NL)
(72) Inventor: Bonrath, Werner, 79115 Freiburg (DE); Letinois, Ulla, 68300 Saint-Louis (FR)
(74) Representative: Steck, Melanie

(57) **Abstract**

The present invention is directed to a process for the manufacture of a mono-ketone comprising the step of hydrogenating a 1,3-diketone with hydrogen in the presence of a bifunctional catalyst having an acid functionality and a hydrogenation functionality.

## Description

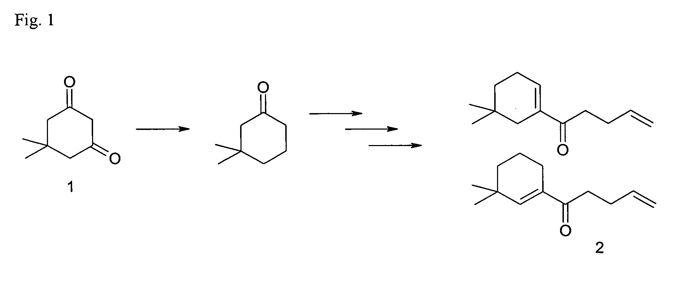

The present invention relates to the synthesis of ketones from 1,3-diketones by hydrogenation in the presence of a bifunctional catalyst. The present invention especially relates to the synthesis of 3,3-dimethylcyclohexanone by hydrogenation of dimedone (5,5-dimethylcyclohexane-1,3-dione) in the presence of a bifunctional catalyst. The latter is the first step in a multi-step synthesis of green ketone (1- (3,3-dimethylcyclohex-6-en-1-yl)-pent-4-en-1-one and 1- (3,3-dimethylcyclohex-1-en-1-yl)-pent-4-en-1-one) (see Fig. 1: dimedone = 1; green ketone = 2).

Thus, 3,3-dimethylcyclohexanone is an important intermediate for the synthesis of green ketone. Green ketone is an important ingredient for the perfumery industry and can be used as perfuming and/or odor-modifying agent as well as flavoring and/or taste-modifying ingredient. Since dimedone is a cheap and readily available starting material, a multi-step synthesis of green ketone starting from dimedone via 3,3-dimethylcyclohexanone is very attractive. Such a multi-step synthesis is e.g. described in US 4,147,672. According to US 4,147,672 dimedone is transformed into 3,3-dimethylcyclohex-5-enone by eliminating the phenylhydrazone. The enone is then hydrogenated in the presence of palladium on charcoal to 3,3-dimethylcyclohexan-1-one (no yield given). Main disadvantages are the waste formation and the handling of the phenylhydrazone.

Further prior art with respect to this reaction is represented by J. Champagne et al., Can. J. Chem. 1964, 42, 212-222; Y. Sasson et al., Tetrahedron Lett. 1973, 34, 3199-3202; B.R. Davis et al., J. Chem. Soc. Perkin Trans 1, 1979, 11, 2820-2825; R.A. Cormier, Synth. Commun. 1981, 11(4), 295-298; and A.G. Martinez et al., Tetrahedron 1987, 43(1), 275-279.

J. Champagne et al. obtained 3,3-dimethylcyclohexanone in a two-step synthesis from dimedone which was reduced with Raney-Ni in 72% yield to 3,3-dimethylcyclohexanol which again was oxidized to 3,3-dimethylcyclohexanone in 95.5% yield with dichromate MST / 28.05.2008 in sulphuric acid (overall yield 69%). The disadvantages of this route are: two steps, toxic dichromate and strong corrosive acid.

Y. Sasson et al. achieved reduction of dimedone via transfer hydrogenation in ethylene glycol as hydrogen donor in the presence of the homogenous catalyst RuCl₂(PPh₃)₃. Among a number of different reduction products 3,3-dimethylcyclohexanone was obtained only in 4% yield which is an unacceptable low selectivity.

Clemmensen reduction of dimedone with amalgamated zinc wool according to B.R. Davis et al. provided 3,3-diemethylcyclohexanone in a mixture with 2,4,4-trimethylcyclopentanone and 3-ethoxy-5,5-dimethylcyclohex-2-enone. No yields or further work-up conditions are given. Additional disadvantage is the use of mercury and lack of details for Zn and Hg removal.

R.A. Cormier obtained 3,3-dimethylcyclohexanone in 69-73% yield (98 - 99% purity) by Pd-catalyzed medium-pressure hydrogenation of dimedone in conc. H₂SO₄/propionic acid. The propionate ester of 3,3-dimethylcyclohexanol which was obtained as by-product had to be separated by distillation. The disadvantages of this method are: highly corrosive conditions which should be avoided on large scale, the formation of by-product and only moderate yield.

Finally, A.G. Martinez et al. reduced dimedone in a two-steps procedure with sodium hydride and triflic anhydride via 5,5-dimethyl-3-trifluoromethylsulfonyloxy-cyclohex-2-en-1-one which was then hydrogenated in the presence of PtO₂. No yields are given. The disadvantages of this procedure are the high price and corrosive nature of triflic anhydride, the expensive NaH which is used in stoichiometric amounts, the salt formation and the two steps.

Therefore, it was necessary to find reaction conditions for the reduction of dimedone to 3,3-dimethylcyclohexanone with high selectivity in one step with no or low waste formation and in the absence of strong oxidative (or corrosive) reagents and halides.

In accordance with the present invention it has been found that 3,3-dimethylcyclohexanone can be obtained from dimedone in high yield by hydrogenation in a polar solvent in the presence of a bifunctional catalyst. The bifunctional catalyst is characterized in having an acidic group and a hydrogenation function, i.e. having an acid functionality and a hydrogenation functionality.

The reaction may also be applied to other 1,3-diketones. Thus, the present invention is directed to a process for the manufacture of a mono-ketone comprising the step of hydrogenating a 1,3-diketone with hydrogen in the presence of a bifunctional catalyst. If the 1,3-diketones are liquid at room temperature (20-25°C) and at atmospheric pressure, the hydrogenation reaction may be carried out without solvent. Preferably the hydrogenation reaction is carried out in a polar solvent.

### 1,3-Diketones

The structure of the 1,3-diketones is not critical. The 1,3-diketones should, however, not contain any double C=C or triple C=C bonds since these may be also reduced under the reaction conditions. Preferably the 1,3-diketones contain the structural element C(O)CR²R³C(O) with R² being hydrogen and R³ being either hydrogen, linear C₁₋₆ alkyl or branched C₃₋₆ alkyl.

The 1,3-diketones are preferably of the formula I, II, III or IV:

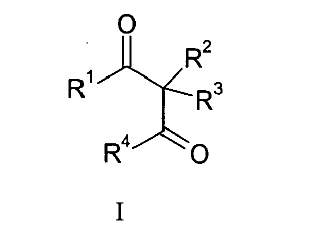

Formula I with R¹ and R⁴ being independently from each other alkyl, aryl or alkylaryl, or with R¹ and R⁴ being together an alkylene,
R² being hydrogen and R³ being either hydrogen, linear C₁₋₆ alkyl or branched C₃₋₆ alkyl; 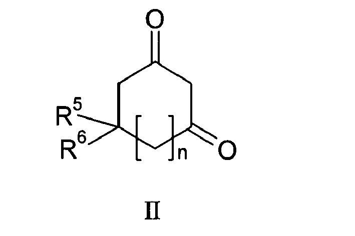 formula II with R⁵ and R⁶ being independently from each other hydrogen, a straight/linear C₁₋₁₂ alkyl (preferably a linear C₁₋₆ alkyl) or a branched C₃₋₁₂ alkyl (preferably a branched C₃₋₆ alkyl), whereby either R⁵ or R⁶ or both may be substituted with hydroxy, linear C₁₋₆ alkoxy (such as methoxy) or branched C₃₋₆ alkoxy (such as iso-propoxy);
and with n being an integer from 0 to 6, preferably with n being 0, 1 or 2, more preferably with n being 0 or 1;
(In case n = 0 the compound of formula II is a 1,3-cyclopentanedione; in case n = 1 the compound of formula II is a 1,3-cyclohexanedione.) 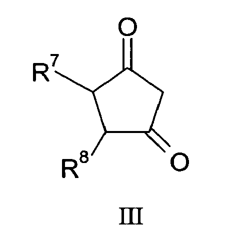 formula III with R⁷ and R⁸ being independently from each other hydrogen, a straight C₁₋₁₂ alkyl (preferably a linear C₁₋₆ alkyl) or a branched C₃₋₁₂ alkyl (preferably a branched C₃₋₆ alkyl), whereby either R⁷ or R⁸ or both may be substituted with hydroxy, linear C₁₋₆ alkoxy (such as methoxy) or branched C₃₋₆ alkoxy (such as iso-propoxy); 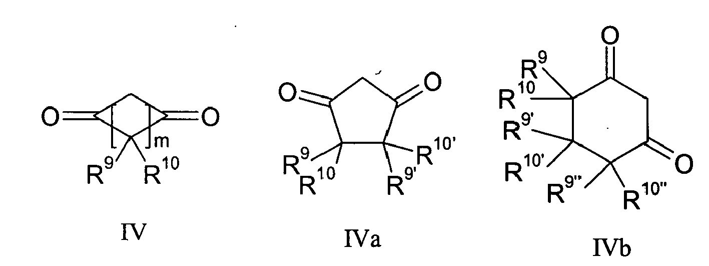 formula IV with R⁹ and R¹⁰ being independently from each other hydrogen, a straight C₁₋₁₂ alkyl (preferably a linear C₁₋₆ alkyl), a branched C₃₋₁₂ alkyl (preferably a branched C₃₋₆ alkyl), whereby either R⁹ or R¹⁰ or both may be substituted with hydroxy, linear C₁₋₆ alkoxy (such as methoxy) or branched C₃₋₆ alkoxy (such as iso-propoxy);
and with m being an integer from 2 to 6, preferably with m being 2, 3 or 4, more preferably with m being 2 or 3 whereby each CR⁹R¹⁰ unit may be equal to or different from the other.

Preferred examples of compounds of formula IV are compounds of formula IVa, i.e. the compounds of formula IV, where m = 2, and compounds of formula IVb, i.e. the compounds of formula IV, where m = 3.

### Substituents R¹ and R⁴ of formula I

The alkyl may contain 1 to 12 C atoms, it may be linear (straight), branched or cyclic and it may be substituted with hydroxy (OH) or alkoxy (OR), whereby R is linear C₁₋₆ alkyl (such as methyl) or branched C₃₋₆ alkyl (such as iso-propyl).

Preferred examples of "aryl" are phenyl (see e.g. compound of formula Ia), naphthyl (see e.g. compound of formula Ic) and pyridyl (see e.g. compound of formula Ib).

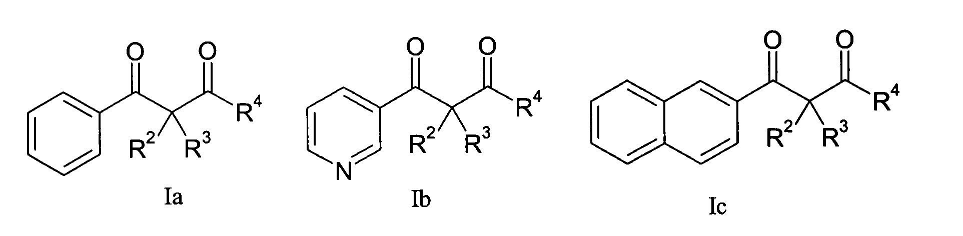

A preferred example of alkylaryl is benzyl (Ph-CH₂).

The term "R¹ and R⁴ being together an alkylene" means that R¹-R⁴ is (CR'R")ₚ with p being an integer from 1 to 6 and each (CR'R") unit being equal to or different from each other, whereby R' and R'' are independently from each other hydrogen or alkyl (preferably linear C₁₋₆ alkyl or branched C₃₋₆ alkyl) or (C₁₋₆) alkyl (linear or branched) substituted with OH or OR with R being linear C₁₋₆ alkyl or branched C₃₋₆ alkyl.

Thus, in the case that R¹ and R⁴ are together alkylene the compound of formula I is a cycloalkane-1,3-dione, which may optionally be substituted with alkyl, whereby said alkyl may further be substituted with hydroxy or alkoxy.

Preferably R¹-R⁴ is (CR'R'')ₚ with p being either 2 or 3 and R' = R'' being either hydrogen or methyl.

The most preferred 1,3-diketone in the context of the present invention is dimedone.

### Bifunctional catalysts

The bifunctional catalyst is characterized in having an acidic group and a hydrogenation function, i.e. having an acid functionality and a hydrogenation functionality.

Preferably the two functions are combined in a resin, so that the bifunctional catalyst used in the process of the present invention is a metal supported acidic resin, i.e. a resin having a hydrogenation function as well as an acidic group.

The acidic group of the catalyst may be represented by sulphonic acid groups (-SO₃H) or by any other group having a pKₐ ≥ 4, preferably the acidic function of the catalyst is represented by sulphonic acid groups. That means that the catalyst is preferably a metal supported polymer/resin with functional sulphonic acid groups. More preferably this catalyst has average particle diameters from 100 µm to 2 mm, preferably from 150 µm to 1.5 mm and more preferably from 250 µm to 1 mm; and a surface area from 10 to 100, preferably from 15 to 75 and more preferably from 20 to 50 square meters/gram (m²/g).

The hydrogenation function of the catalyst may be represented by metals well-known and used for hydrogenations such as e.g. Ni, Pd, Pt, Rh, Ru and Ir, Au, with which the polymeric resin is doped. Thus, the bifunctional catalyst is preferably a polymeric resin having sulphonic acid groups and being doped with a metal selected from the group consisting of nickel, palladium, platinum, rhodium, ruthenium, iridium and gold.

Preferably the hydrogenation function of the catalyst is represented by Pd, Pt or Ni, with which the polymeric resin is doped. More preferably the hydrogenation function of the catalyst is represented by Pd, with which the polymeric resin is doped.

The bifunctional catalyst may contain the metal in an amount of at least 0.1 g / L of the catalyst. With such an amount a reasonable space-time-yield results. Preferably the bifunctional catalyst contains the metal in an amount in the range of from 0.1 to 10 g / L of the catalyst, more preferably in an amount in the range of from 1 to 8 g / L of the catalyst, most preferably in an amount in the range of from 2 to 7 g / L of the catalyst.

The polymeric resin may be a styrene-divinylbenzene copolymer, a divinylbenzene polymer, any divinylbenzene copolymer, any styrene copolymer or any other polymer to which sulphonic acid groups may be attached.

In a preferred embodiment of the present invention the bifunctional catalyst is a metal-doped polysulfonated ion exchange resin catalyst as described and disclosed in EP-A 1 321 450. Preferably, the polysulfonated cation exchange resin catalysts are in the form of macroporous spherical beads having average particle diameters from 100 µm to 2 mm, preferably from 150 µm to 1.5 mm and more preferably from 250 µm to 1 mm; have a sulfonic acid group content of 5.0 to 7.0, preferably 5.1 to 6.5 and more preferably 5.2 to 6.0 meq/g, based on dry weight of polysulfonated cation exchange resin; loaded with 0.1 to 10, preferably 1 to 8 and more preferably 2 to 7, g of metal or metal ion per L of the catalyst, based on dry weight of polysulfonated cation exchange resin; typically possess a surface area from 10 to 100, preferably from 15 to 75 and more preferably from 20 to 50 square meters/gram (m²/g); and a total porosity in the range of 0.1 to 0.9, preferably in the range of 0.2 to 0.7 and more preferably in the range of from 0.25 to 0.5 cubic centimeter pores per gram polymer (cm³/g), with an average pore diameter of 50 to 2,500 Ångstrom units and preferably 150 to 1000 Ångstrom units. Porosities are defined according to IUPAC (International Union of Pure and Applied Chemistry) nomenclature as follows:
Microporosity = pores less than 20 Ångstrom units
Mesoporosity = pores between 20 and 500 Ångstrom units
Macroporosity = pores greater than 500 Ångstrom units

In a further preferred embodiment of the present invention the bifunctional catalyst is a macroreticular ion exchange resin of styrene-divinylbenzene copolymer in bead form having sulphonic acid groups and being doped with a metal selected from the group consisting of nickel, palladium, platinum, rhodium, ruthenium, iridium and gold (most preferably this metal is palladium).

Such copolymers are commercially available from Rohm & Haas (Springhouse, Philadelphia, USA) as e.g. Amberlyst CH28. Amberlyst CH28 is a bead form, macroreticular, sulphonic acid, palladium doped ion exchange resin having a concentration of the acid sites (i.e. the sulphonic acid groups) ≥ 1.6 eq/L and ≥ 4.8 eq/kg, a harmonic mean particle size in the range as from 0.850 to 1.050 mm, a uniformity coefficient ≤ 1.4, a surface area of 33 m²/g (measured by nitrogen BET), an average pore diameter of 240 Å and a total pore volume of 0.20 ml/g. These copolymers may be loaded with the metal as defined above in an amount in the range of from 0.1 to 10 g / L of the catalyst, preferably in an amount in the range of from 1 to 8 g / L of the catalyst, more preferably in an amount in the range of from 2 to 7 g / L of the catalyst.

A weight ratio of catalyst to 1,3-diketone, especially to dimedone, in the range of (0.2 g - 1.0 g) to 1.0 g is sufficient for very good results.

In the most preferred embodiment of the present invention the catalyst Amberlyst CH57 is used. This catalyst is a bead form, macroreticular, sulphonic acid, palladium doped ion exchange resin having a concentration of the acid sites (i.e. the sulphonic acid groups) ≥ 1.6 eq/L and ≥ 4.8 eq/kg, a harmonic mean particle size in the range as from 0.850 to 1.050 mm, a uniformity coefficient ≤ 1.4, a surface area of 33 m²/g (measured by nitrogen BET), an average pore diameter of 240 Å and a total pore volume of 0.20 ml/g and it contains 5.7 g Pd /L Amberlyst, which corresponds to 5.7 g palladium/ 700 g Amberlyst. Here the preferred weight ratio of Amberlyst CH57 to 1,3-diketone, especially to dimedone, is in the range of (0.2 g - 1.0 g) to 1.0 g.

### Solvents

Among the polar solvents in which the reaction can be conducted are, without limitation, water, alcohols, ethers (cyclic and open chain) and alkyl esters of organic acids. Mixtures of these solvents may also be used.

Preferred are alcohols having from 1-4 carbon atoms (C₁₋₄ alcohols), i.e. methanol, ethanol, propanol, iso-propanol, butanol, 2-butanol, tert-butanol and 2-methylpropanol. Most preferred are methanol, ethanol and isopropanol.

Examples of ethers (cyclic and open chain) are diethyl ether, diisopropyl ether and tetrahydrofuran.

Examples of alkyl esters of organic acids are ethyl acetate, butyl acetate and methyl acetate.

The 1,3-diketone (especially dimedone) may be hydrogenated according to the process of the present invention as a 10 - 40 weight-% solution, preferably as a 15 - 35 weight-% solution, in one or more of these solvents, preferably in C₁₋₄ alcohols, more preferably in methanol, ethanol or iso-propanol, most preferred in methanol. That means the weight ratio of the 1,3-diketone to the polar solvent is in the range of from 0.1 - 0.4 to 1, preferably in the range of from 0.15 - 0.35 to 1.

### Reaction conditions

The process according to the present invention may be carried out at a temperature in the range of from 20 to 130°C, preferably in the range of from 50 to 110 °C, more preferably in the range of from 50 to 100°C, most preferably in the range of from 60 to 100°C **and** at a hydrogen pressure in the range of 1 - 20 bara, preferably in the range of 1 - 15 bara, more preferably in the range of 1.1 - 10 bara. The unit "bara" means "bar absolute" and is a technical number.

The optimum solvent is dependent on the nature of the 1,3-diketone and the catalyst, the optimum temperature and pressure is dependent on all of them (i.e. solvent, 1,3-diketone and catalyst).

Preferably the reaction mixture is stirred, more preferably the velocity with which the reaction mixture is stirred lies in the range of from 250 rpm (rounds per minute) to 1000 rpm. Without stirring the reaction times are longer and the selectivity is worse compared to carrying out the reaction with stirring.

It could be shown that the bifunctional catalyst leads to high yield and selectivity in the hydrogenation of 1,3-diketones (especially dimedone). This yield and selectivity remains high when the bifunctional catalyst is used several times. Furthermore, the amount of metal is reduced compared to common hydrogenation systems such as Pd on charcoal.

### PREFERRED EMBODIMENT OF THE PRESENT INVENTION

The preferred embodiment of the present invention is a process for the manufacture of 3,3-dimethylcyclohexanone comprising the step of hydrogenating dimedone with hydrogen in the presence of a bifunctional catalyst having an acid functionality and a hydrogenation functionality in a polar solvent.

The polar solvent is preferably methanol, ethanol, isopropanol or any mixture thereof.

The bifunctional catalyst is preferably a metal supported polymer/resin with functional sulphonic acid groups, which is doped with Ni, Pd, Pt, Rh, Ru, Ir or Au or any mixture thereof, preferably it is doped with Pd, Pt, Ni or any mixture thereof; more preferably it is doped with Pd.

Preferably the bifunctional catalyst contains the metal (i.e. Ni, Pd, Pt, Rh, Ru, Ir or Au or any mixture thereof) in an amount in the range of from 0.1 to 10 g / L of the catalyst, more preferably in an amount in the range of from 1 to 8 g / L of the catalyst, most preferably in an amount in the range of from 2 to 7 g / L of the catalyst.

The polymeric resin may be a styrene-divinylbenzene copolymer or a divinylbenzene polymer or any other polymer to which sulphonic acid groups may be attached. Preferably the bifunctional catalyst is a macroreticular ion exchange resin of styrene-divinylbenzene copolymer in bead form having sulphonic acid groups and being doped with a metal selected from the group consisting of nickel, palladium, platinum, rhodium, ruthenium, iridium and gold. Most preferably this metal is palladium. Preferably the concentration of the acid sites (i.e. the sulphonic acid groups) of those polymers is ≥ 1.6 eq/L and ≥ 4.8 eq/kg, and/or the harmonic mean particle size is in the range as from 0.850 to 1.050 mm, and/or the surface area is in the range of from 10 to 100 (preferably 15 - 75, more preferably 20 - 50) m²/g (measured by nitrogen BET) and/or the average pore diameter is in the range of from 50 to 2500 Å (preferably in the range of from 150 to 1000 A). These polymers may be loaded with the metal as defined above in an amount in the range of from 0.1 to 10 g / L of the catalyst, preferably in an amount in the range of from 1 to 8 g / L of the catalyst, more preferably in an amount in the range of from 2 to 7 g / L of the catalyst.

A weight ratio of catalyst to dimedone in the range of (0.2 g - 1.0 g) to 1.0 g is sufficient for very good results.

In the most preferred embodiment of the present invention the catalyst Amberlyst CH57 as defined above is used. This catalyst contains 5.7 g Pd /L Amberlyst, which corresponds to 5.7 g palladium/ 700 g Amberlyst. Here the preferred weight ratio of Amberlyst CH57 to dimedone is in the range of (0.2 g - 1.0 g) to 1.0 g.

The hydrogenation of dimedone to 3,3-dimethylcyclohexanone is preferably carried out at a temperature in the range of from 20 to 130°C, preferably in the range of from 50 to 110 °C, more preferably in the range of from 50 to 100°C, most preferably in the range of from 60 to 100°C **and** at a hydrogen pressure in the range of 1 - 20 bara, preferably in the range of 1 - 15 bara, more preferably in the range of 1.1 - 10 bara.

Preferably the dimedone is hydrogenated as a 10 - 40 weight-% solution, preferably as a 15 - 35 weight-% solution, in one or more of the solvents as cited above, preferably in C₁₋₄ alcohols, more preferably in methanol, ethanol or iso-propanol, most preferably in methanol.

Preferably the reaction mixture is stirred, more preferably the velocity with which the reaction mixture is stirred lies in the range of from 250 rpm (rounds per minute) to 1000 rpm. Without stirring the reaction times are longer and the selectivity is worse compared to carrying out the reaction with stirring.

It could be shown that the bifunctional catalyst leads to high yield and selectivity in the hydrogenation of dimedone. This yield and selectivity remains high when the bifunctional catalyst is used several times. Furthermore, the amount of metal is reduced compared to common hydrogenation systems such as Pd on charcoal.

The following non-limiting examples illustrate the invention in more detail.

### Examples

In all the examples 1 to 7 and 4-1 to 4-4 according to the process of the present invention the catalyst Amberlyst CH57 was used. This catalyst contains 5.7 g Pd /L Amberlyst, which corresponds to 5.7 g palladium/ 700 g Amberlyst. Furthermore it is a bead form, macroreticular, sulphonic acid, palladium doped ion exchange resin having a concentration of the acid sites (i.e. the sulphonic acid groups) ≥ 1.6 eq/L and ≥ 4.8 eq/kg, a harmonic mean particle size in the range as from 0.850 to 1.050 mm, a uniformity coefficient ≤ 1.4, a surface area of 33 m²/g (measured by nitrogen BET), an average pore diameter of 240 Å and a total pore volume of 0.20 ml/g.

### The following abbreviations were used:

MeOH = methanol, EtOH = ethanol, iPrOH = iso-propanol.

### General procedure for examples 1-7:

Amberlyst CH57 (1 g cat per g dimedone; = 1 mol% Pd in relation to dimedone) was placed in a glass-liner. 4.38 g (31 mmol) of dimedone and ca. 15 g of the solvent (leading to a 20 weight-% solution of dimedone) were added. The glass-liner was closed and stirring was started. The autoclave was flushed three times with 5 bara N₂. The stirrer was turned off. The autoclave was pressurized with 5 bara H₂ (i.e. 1 bar atmospheric pressure + 4 bar H₂ pressure) for 10 minutes for pressure check. The pressure was released. The stirrer was turned on again and the autoclave was heated to 85°C internal temperature. The autoclave was pressurized with H₂ and the stirrer was set on. The reaction mixture was stirred under H₂-pressure at 85°C until hydrogen uptake had ceded (when the hydrogenation curve is horizontal, usually after 110% of theoretical H₂-amount). Then the autoclave was cooled to a temperature below 25°C and depressurized. After three times flushing with N₂ at 5 bara for 10 minutes stirring was stopped and the autoclave was opened. The content was sucked and filtrated over a 0.45 µm filter. A 30 µL filtrated sample was diluted with 1 mL of isopropanol and 5 mg of NaHCO₃ and then analyzed by GC.

**Table 1: Use of Amberlyst CH57 as catalyst**

| **Example** | **Solvent** | **H₂-Pressure [bar]** | **Time [h]** | **Yield [%]** | **Selectivity [%]** |
|---|---|---|---|---|---|
| **1** | MeOH | 2 | 17 | 96.7 | 98.6 |
| **2** | MeOH | 2 | 30.8 | 96 | 97 |
| **3*** | MeOH | 2 | 7.5 | 98 | 98 |
| **4-1** | MeOH | 10 | 5 | 94.5 | 97 |
| **4-1** | MeOH | 10 | 7 | 94.5 | 96.4 |
| **5** | EtOH | 2 | 22 | 94.0 | 94 |
| **6** | EtOH | 10 | 5 | 94.7 | 95 |
| **7** | iPrOH | 10 | 16 | 91.4 | 93 |

### * Example 3: Exp. 20804 Hydrogenation in methanol at a larger scale

37 g of Amberlyst CH57 were placed in a 500 mL glass-liner. 32 g of dimedone (221 mmol) and 116 g of methanol (147 mL) were added. The glass-liner was closed and stirring was started with 500 rpm. The autoclave was flushed three times with 5 bara N₂. The stirrer was turned off. The autoclave was pressurized with 5 bara H₂ for 10 minutes for pressure check. The pressure was released. The stirrer was turned on to 1000 rpm and the autoclave was heated to 85°C internal temperature. The autoclave was pressurized with 2 bara H₂ and the stirrer was set to 1000 rpm. The reaction mixture was stirred under 2 bara H₂ at 85°C for 7.5 hours. Then the autoclave was cooled to a temperature below 25°C and depressurized. After three times flushing with N₂ at 5 bara for 10 minutes stirring was stopped and the autoclave was opened. The content was sucked and filtrated over a 0.45 µm filter. A 30 µL filtrated sample was diluted with 1 mL of isopropanol and 5 mg of NaHCO₃ and then analyzed by GC. The total yield of 3,3-dimethylcyclohexanone was 98% with a selectivity of 98 %.

Here 37 g of Amberlyst CH57; 32 g of dimedone (221 mmol) and 116 g of methanol (147 mL) were used showing that the process of the present invention may also be successfully, i.e. with high yield and selectivity, carried out at a larger scale.

### Example 4-1 to 4-4: Hydrogenation in methanol with catalyst recycling

4.99 g of Amberlyst CH57 were placed in a 37 mL glass-liner. 4.38 g of dimedone (31 mmol) and 15.6 g of methanol (19.7 mL) were added. The glass-liner was closed and stirring was started with 500 rpm. The autoclave was flushed three times with 5 bara N₂. The stirrer was turned off. The autoclave was pressurized with 5 bara H₂ for 10 minutes for pressure check. The pressure was released. The stirrer was turned on again to 1000 rpm and the autoclave was heated to 85°C internal temperature. The autoclave was pressurized with 10 bara H₂ and the stirrer was set to 1000 rpm. The reaction mixture was stirred under 10 bara H₂ at 85°C for 7 hours. Then the autoclave was cooled to a temperature below 25°C and depressurized. After three times flushing with N₂ at 5 bara for 10 minutes stirring was stopped and the autoclave was opened. The content was sucked and filtrated over a 0.45 µm filter. A 30 µL filtrated sample was diluted with 1 mL of isopropanol and 5 mg of NaHCO₃ and then analyzed by GC. The total yield of 3,3-dimethylcyclohexanone was 96% with a selectivity of 97 %.

The catalyst was immediately placed again in a 37 mL glass liner and the same procedure (example 4) was repeated with fresh dimedone and methanol (example 4-2). The yield of 3,3-dimethylcyclohexanone was 96% with a selectivity of 97%. This was again repeated twice (see the results in table 2). The selectivity remained superior to 90% for 4 cycles. The yield in the second cycle was superior to 95%. Though the yield was rather low in cycle 3 and 4, the non-reacted starting material could be recycled so that there was no loss in starting material.

**Table 2: Recycling experiments with Amberlyst CH57 as catalyst**

| **Example** | **Solvent** | **H₂-Pressure [bara]** | **Time [h]** | **Yield [%]** | **Selectivity [%]** |
|---|---|---|---|---|---|
| **4-1** | MeOH | 10 | 5 | 94.5 | 97 |
| **4-2** | MeOH | 10 | 7 | 96.3 | 97 |
| **4-3** | MeOH | 10 | 7.3 | 47 | 99 |
| **4-4** | MeOH | 10 | 17.3 | 30 | 99 |

### Example 7: Hydrogenation in isopropanol at 10 bara

4.99 g of Amberlyst CH57 were placed in a 37 mL glass-liner. 4.38 g of dimedone (31 mmol) and 15.6 g of isopropanol (20 mL) were added. The glass-liner was closed and stirring was started with 500 rpm. The autoclave was flushed three times with 5 bara N₂. The stirrer was turned off. The autoclave was pressurized with 5 bara H₂ for 10 minutes for pressure check. The pressure was released. The stirrer was turned on again to 1000 rpm and the autoclave was heated to 85°C internal temperature. The autoclave was pressurized with 10 bara H₂ and the stirrer was set to 250 rpm. The reaction mixture was stirred under 10 bara H₂ at 85°C for 16 hours. Then the autoclave was cooled to a temperature below 25°C and depressurized. After three times flushing with N₂ at 5 bara for 10 minutes stirring was stopped and the autoclave was opened. The content was sucked and filtrated over a 0.45 µm filter. A 30 µL filtrated sample was diluted with 1 mL of isopropanol and 5 mg of NaHCO₃ and then analyzed by GC. The total yield of 3,3-dimethylcyclohexanone was 91 % with a selectivity of 93 %.

### Comparison examples C1 to C4-2

### Examples C1, C2, C3: Use of Pd/C as catalyst

Conditions with Pd/C as catalyst: 4.38 g (31 mmol) of dimedone in 15 g of solvent (20 weight-%), 620 mg of catalyst / 4.38 g dimedone (= 2 mol% Pd in relation to dimedone), 85°C, hydrogenation until hydrogen uptake has ceded (when the hydrogenation curve is horizontal, usually after 110% of theoretical H₂-amount).

The reaction over Pd/C leads only to selectivities of 90% or higher when the hydrogenation is carried out in at least catalytic amounts of acid like pTsOH (example C2). When the acid is absent, selectivities are lower than 90%. When Pd/C is washed prior to use, so that acid traces from the Pd/C preparation are washed away, the yield decreases even to 47% and the selectivity to 67% (example C1). In the absence of acid, the hydrogenation only yields 90% yield with a selectivity of 90% (example C3).

### Advantages when using Amberlyst CH57 compared to Pd/C:

The initial amount of Pd can be reduced when using Amberlyst CH57, the yield and selectivity when using the Amberlyst CH57 are higher with 1 mol% Pd compared to Pd/C using at least 2 mol% Pd.

**Table 3: Use of Pd/C as catalyst**

| **Comparison Example** | **Conditions** | **Time [h]** | **Yield [%]** | **Selectivity [%]** |
|---|---|---|---|---|
| **C1** | MeOH, 2 bar H₂, catalyst pre-washed | 31 | 47 | 67 |
| **C2** | MeOH, pTsOH (2 mol%), 2 bar H₂ | 2 | 95 | 95 |
| **C3** | MeOH, 2 bar H₂ | 2 | 90.4 | 91 |

### Example C4-1 & C4-2: Recycling experiments

4.38 g (31 mmol) of dimedone in 15 g of solvent (20 wt%), 1 g of catalyst /g of dimedone (= 1 mol% Pd in relation to dimedone), 85°C, hydrogenation until hydrogen uptake has ceded (when the hydrogenation curve is horizontal, usually after 110% of theoretical H₂-amount). After the experiment, the reaction mixture was filtrated under inert atmosphere and the wet catalyst was used for the next cycle.

When Pd/C is used as hydrogenation catalyst, recycling experiments did not work well: The selectivity dropped in the second cycle to 60% and the yield to 31 % whereas using Amberlyst CH57, the selectivity remained superior to 90% for 4 cycles and the yield in the second cycle was superior to 95%.

**Table 4: Recycling experiments with Pd/C as catalyst**

| Exp. number | **Example** | **Solvent** | **H₂-Pressure [bara]** | **Time [h]** | **Yield [%]** | **Selectivity [%]** |
|---|---|---|---|---|---|---|
| 20681-1 | **C4-1** | MeOH | 2 | 2 | 90 | 90 |
| 20681-2 | **C4-2** | MeOH | 2 | 17 | 31 | 64 |

With Pd/C the best selectivity was reached in the first cycle with 2 bar H₂-pressure, this is the reason for the lower pressure in the recycling experiments C4-1 and C4-2 compared to examples 4-1 to 4-4.

## Claims

1. A process for the manufacture of a mono-ketone comprising the step of hydrogenating a 1,3-diketone with hydrogen in the presence of a bifunctional catalyst having an acid functionality and a hydrogenation functionality.

2. The process according to claim 1, wherein the hydrogenation reaction is carried out in a polar solvent.

3. The process according to claim 1 or 2, wherein the 1,3-diketone is a compound of the formula I 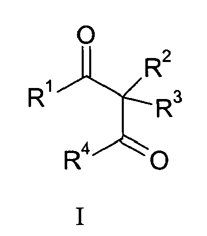
with R¹ and R⁴ being independently from each other alkyl, aryl or alkylaryl, or with R¹ and
R⁴ being together an alkylene,
R² being hydrogen and R³ being either hydrogen, linear C₁₋₆ alkyl or branched C₃₋₆ alkyl.

4. The process according to claim 1, wherein the 1,3-diketone is a compound of the formula II 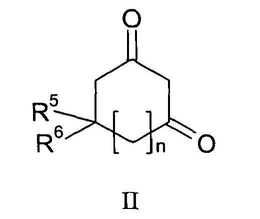
with R⁵ and R⁶ being independently from each other hydrogen, a linear C₁₋₁₂ alkyl or a branched C₃₋₁₂ alkyl, whereby either R⁵ or R⁶ or both may be substituted with hydroxy, linear C₁₋₆ alkoxy or branched C₃₋₆ alkoxy;
and with n being an integer from 0 to 6, preferably with n being 0, 1 or 2, more preferably with n being 0 or 1.

5. The process according to claim 1, wherein the 1,3-diketone is a compound of the formula III 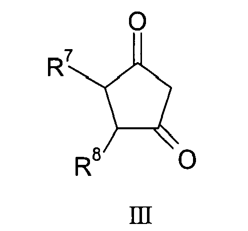
with R⁷ and R⁸ being independently from each other hydrogen, a straight C₁₋₁₂ alkyl or a branched C₃₋₁₂ alkyl, whereby either R⁷ or R⁸ or both may be substituted with hydroxy, linear C₁₋₆ alkoxy or branched C₃₋₆ alkoxy.

6. The process according to claim 1, wherein the 1,3-diketone is a compound of the formula IV 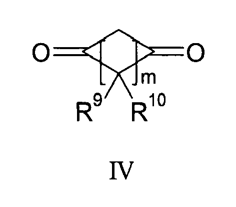
with R⁹ and R¹⁰ being independently from each other hydrogen, a straight C₁₋₁₂ alkyl or a branched C₃₋₁₂ alkyl, whereby either R⁹ or R¹⁰ or both may be substituted with hydroxy, linear C₁₋₆ alkoxy or branched C₃₋₆ alkoxy;
and with m being an integer from 2 to 6, preferably with m being 2, 3 or 4, more preferably with m being 2 or 3, whereby each CR⁹R¹⁰ unit may be equal to or different from the other.

7. The process according to claim 2, wherein the mono-ketone is 3,3-dimethylcyclohexanone and the 1,3-diketone is dimedone.

8. The process according to any of the preceding claims wherein the bifunctional catalyst is a polymeric resin having sulphonic acid groups and being doped with a metal selected from the group consisting of nickel, palladium, platinum, rhodium, ruthenium, iridium and gold.

9. The process according to claim 8 wherein the bifunctional catalyst is a macroretinal ion exchange resin of styrene-divinylbenzene copolymer in bead form having sulphonic acid groups and being doped with a metal selected from the group consisting of nickel, palladium, platinum, rhodium, ruthenium, iridium and gold.

10. The process according to claim 8 or 9 wherein the metal with which the resin is doped is palladium.

11. The process according to one or more of claims 1 to 7 wherein the bifunctional catalyst is a metal supported acidic resin, whereby the metal is selected from the group consisting of nickel, palladium, platinum, rhodium, ruthenium, iridium and gold.

12. The process according to any of the preceding claims wherein the polar solvent is selected from the group consisting of water, alcohols, ethers, alkyl esters of organic acids and mixtures thereof.

13. The process according to any of the preceding claims wherein the hydrogenation is carried out at a temperature in the range of from 20 to 130°C and at a hydrogen pressure in the range of from 1 to 20 bar absolute.

14. The process according to any of the preceding claims wherein the weight ratio of the 1,3-diketone to the polar solvent is in the range of from 0.1 - 0.4 to 1.

15. The process according to any of the preceding claims wherein the weight ratio of the bifunctional catalyst to the 1,3-diketone is in the range of from 0.2 - 1.0 g to 1.0 g.
